(19) Europäisches Patentamt
European Patent Office
Office européen des brevets

(11) Publication number: **0 354 453 B1**

# (12) EUROPEAN PATENT SPECIFICATION

(45) Date of publication of patent specification: **25.01.95**

(51) Int. Cl.6: **A61K 31/535**, //C07D498/06, (C07D498/06,265:00,221:00)

(21) Application number: **89114219.2**

(22) Date of filing: **01.08.89**

The file contains technical information submitted after the application was filed and not included in this specification

## (54) Antimicrobial agent for animals.

(30) Priority: **09.08.88 JP 198199/88**

(43) Date of publication of application:
**14.02.90 Bulletin 90/07**

(45) Publication of the grant of the patent:
**25.01.95 Bulletin 95/04**

(84) Designated Contracting States:
**DE ES FR GB GR IT NL**

(56) References cited:
**EP-A- 0 047 005**
**EP-A- 0 206 283**

**PATENT ABSTRACTS OF JAPAN vol. 10, no. 31 (C-327) 06 February 1986, & JP-A-60 184014 (DAIICHI SEIYAKU K.K.) 19 September 1985.**

**CHEMICAL AND PHARMACEUTICAL BULLETIN, vol. 32, no. 12, 1984, pages 4907 - 4913; I. HAYAKAWA et al.: "Synthesis and Antibacterial Activities of Substituted 7-Oxo-2,3-dihydro-7H-pyrido[1,2,3-de ][1,4]benzoxazine-6-carboxylic Acids"**

(73) Proprietor: **DAIICHI PHARMACEUTICAL CO., LTD.**
**14-10, Nihonbashi 3-chome**
**Chuo-ku,**
**Tokyo 103 (JP)**

(72) Inventor: **Takahata, Toshihiro Daiichi Pharmaceutical**
**Research Institute**
**1-16-13, Kitakasai**
**Edogawa-ku**
**Tokyo (JP)**
Inventor: **Takei, Masakazu Daiichi Pharmaceutical**
**Research Institute**
**1-16-13, Kitakasai**
**Edogawa-ku**
**Tokyo (JP)**
Inventor: **Kato, Masahiro Daiichi Pharmaceutical**
**Research Institute**
**1-16-13, Kitakasai**
**Edogawa-ku**
**Tokyo (JP)**

EP 0 354 453 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Miura, Tadayoshi Daiichi Pharmaceutical**
**Research Institute**
**1-16-13, Kitakasai**
**Edogawa-ku**
**Tokyo (JP)**
Inventor: **Yoshioka, Toshiyuki Daiichi Pharmaceutical**
**Research Institute**
**1-16-13, Kitakasai**
**Edogawa-ku**
**Tokyo (JP)**

(74) Representative: **Wächtershäuser, Günter, Prof. Dr.**
**Patentanwalt**
**Tal 29**
**D-80331 München (DE)**

## Description

### BACKGROUND OF THE INVENTION

#### Field of the Invention:

This invention relates to a use of a 3S-type compound selected from pyrido[1,2,3-de][1,4]benzoxazine derivatives represented by the formula (I):

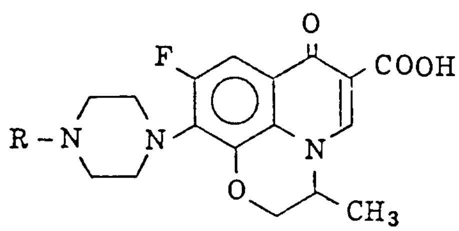

(I)

wherein R is a $C_{1-6}$ alkyl group, or a salt or a hydrate thereof, for the prevention, cure, or treatment of various infectious diseases of animals.

#### Description of the Background:

The increased number of livestock and poultry confined to a unit area by the current stockbreeding practices gives rise to proliferation of various infectious disease among farm animals and domestic birds. Damage caused by such infectious diseases has been increasing yearly, posing an economic problem to the industry.

Conventionally, antibiotics such as tylosin, oxytetracycline, etc., and synthetic antimicrobial agents such as oxolinic acid, piromidic acid, etc. have been added to feed or water, orally administered, or injected to animals for the prevention and cure of their infectious diseases.

These conventional antimicrobial agents, however, have drawbacks such as insufficient antimicrobial activity, a narrow antimicrobial spectrum, development of microbial resistance to the agents, low disease-curing effect, inadequate safety, side effects, high production costs, and the like.

Among pyrido[1,2,3-de][1,4]benzoxazine derivatives of the above formula (I) a compound which is a racemate and has no substituted group at the 3-position in formula (I) has been reported in Japanese Patent Laid-open No. 184014/1985. Japanese Patent Laid-open No. 116217/1984 discloses antimycoplasmal activity of ofloxacin. These compound, however, possess only a narrow antimicrobial spectrum, and their antimicrobial activity is not necessarily sufficient.

EP-A-0 047 005 describes a use of a racemate of a compound having the above general formula (I). From EP-A-0 206 283, it is known that 3S-type compounds of formula (I) posess a higher anti-microbial actvity than the corresponding racemate or 3R-type compound.

It has been found that among the compounds of general formula I

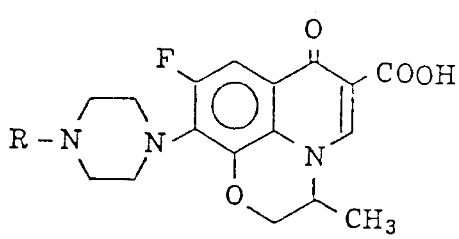

(I)

(wherein R is a $C_{1-6}$ alkyl group), the compound 3S-9-fluoro-2,3-dihydro-3-methyl-10-(4-ethyl-1-piperazinyl)-7-oxo-7H-pyrido [1,2,3-de][1,4]benzoxazine-6-carboxylic acid or a salt or a hydrate thereof exhibited a superior preventive or curing effect against a wide variety of infectious diseases of animals with no or little toxicity. Such a finding has led to the completion of this invention.

## SUMMARY OF THE INVENTION

Accordingly, the present invention provides a use of 3S-9-fluoro-2,3-dihydro-3-methyl-10-(4-ethyl-1-piperazinyl)-7-oxo-7H-pyrido[1,2,3-de][1,4]benzoxazine-6-carboxylic acid or a salt or a hydrate thereof, in the manufacture of a medicament for treating or preventing infectious diseases of animals.

## DETAILED DESCRIPTION OF THE INVENTION AND PREFERRED EMBODIMENTS

The Compound defined in claim 1 can be used as acid addition salts owing to the basic nature of the piperazinyl group of the 10-position. Examples of acid addition salts include inorganic salts such as hydrochloride, hydrobromide, hydroiodide, sulfate, etc. and organic salts such as acetate, methanesulfonate, citrate, benzenesulfonate, lactate, etc.

The carboxy group at the 6-position may take a salt form including alkali metal salts such as sodium salt, potassium salt, etc., alkaline earth metal salts such as magnesium salt, calcium salt, etc., ammonium salts, and organic salts such as triethylamine salt, etc.

Furthermore, salts of said Compound can be used in hydrate forms.

For the preparation of racemates of Compound (I) the methods described in Japanese Patent Laid-open Nos. 46986/1982 and 72589/1983, for example, can be used. The method described in Japanese Patent Laid-open No. 252790/1987 can be given as an example of the method for preparing 3S-type compounds.

The Compound defined in claim 1, their salts or hydrates [hereinafter collectively referred to as Active Compounds (I)] can be dosed to animals by oral administration, as are or mixed with feed. Alternatively, they can be dissolved in water and the solution is orally administered as is, mixed with feed, or further diluted with water. Injection is another method for administrating the Active Compounds (I).

The dose depend upon the purpose of the administration, e.g., prevention, cure, etc., kinds and body weight of animals to be treated, types of microorganisms causing the infection, the degree of infection, and the like. In general, 1-200 mg per day, preferably 5-100 mg per day, is dosed once or several times a day. These ranges are, however, an approximate standard, and it is possible to dose Active Compound (I) in an amount outside the above range, depending on the age, body weight, and degree of disease of the animal. There are no specific limitations as to the period during which Active Compound (I) is administered to animals. Usually, the administration for a period of 1-10 days gives a sufficient result. Intermittent administration is also possible.

Active Compound (I) can be dosed to animals in various forms of preparations that can be prepared according to conventional methods such as powders, subtilized granules, solubilizable powders, syrups, solutions, injections, and the like. Hereinafter are presented typical formulations comprising Active Compound (I).

Formulation Example 1

| Preparation for mixing with feed | |
|---|---|
| Components | Amount (parts by weight) |
| Active Compound (I) | 1-10 |
| Corn starch | 98.5-89.5 |
| Light anhydrous silicic acid | 0.5 |
| Total | 100 |

Formulation Example 2

| Preparation for mixing with feed or water | |
|---|---|
| Components | Amount (parts by weight) |
| Active Compound (I) (Water soluble)<br>Lactic acid | 1-10<br>90-99 |
| Total | 100 |

Formulation Example 3

| Liquid preparation | |
|---|---|
| Components | Amount (parts by weight) |
| Active Compound (I)<br>Acetic acid or sodium hydroxide<br>Ethyl parabenzoate<br>Purified water | 1-10<br>5-20<br>0.1<br>69.6-93.9 |
| Total | 100 |

Antimicrobial agents comprising the active compound used in accordance with the present invention exhibit a wide spectrum of activity against various bacteria causing infectious diseases of animals. They exhibit strong activities against bacteria belonging to genera, for example, of *Escherichia*, *Salmonella*, *Pasteurella*, *Haemophilus*, *Bordetella*, *Staphylococcus*, *Mycoplasma*, etc. They are therefore useful for the prevention, cure, and treatment of infectious diseases of cattle, pigs, birds, dogs, cats, and the like. Named as specific infectious diseases against which the antimicrobial agent of this invention is effective are; for diseases of cattle, *E. coli* infection, *Salmonella* infection, *Mycoplasma* infection, hemorrhagic septicemia, bovine contiguous pleuropneumonia, mastitis, etc.; for diseases of pigs, *E. coli* infection, *Salmonella* infection, *Pasteurella* infection, *Mycoplasma* infection, atrophic rhinitis, exudative epidermitis, etc.; for diseases of birds, *E. coli* infection, chicken pullorum, paratyphoid, bird cholera, infectious coryza, staphylococcus infection, *Mycoplasma* infection, etc.; for diseases of dogs, *E. coli* septicemia, *Salmonella* infection, hemorrhagic septicemia, uterus empyema, cystitis, etc.; and for diseases of cats, pleurisy, cystitis, *Haemophilus* infection, diarrhea, *Mycoplasma* infection, etc.

EXAMPLES

In the following examples Active Compounds listed in Table 1 below were prepared or used.

TABLE 1

Formula:

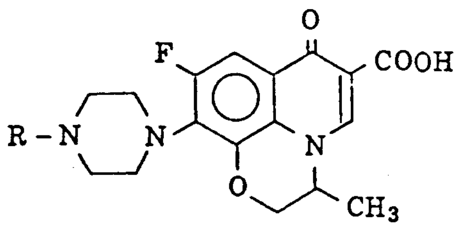

(I)

| Compound Nos. | R in formula (I) | Types |
|---|---|---|
| (1)* | $CH_3$- | 3S-type compound, Hemihydrate |
| (2)* | $C_2H_5$- | 3(RS)-type compound, (Racemate) |
| (3)* | n-$C_3H_7$- | 3(RS)-type compound, (Racemate) |
| (4)* | $(CH_3)_2CH$- | 3(RS)-type compound, (Racemate) |
| (5)* | n-$C_4H_9$- | 3(RS)-type compound, (Racemate) |
| (6) | $C_2H_5$- | 3S-type compound |
| (7)* | n-$C_3H_7$- | 3S-type compound |
| (8)* | $C_2H_5$- | 3(RS)-type compound, (Racemate) Hemihydrate hydrochloride |

*) Compounds 1 to 5, 7 and 8 are provided for comparison purposes only.

Example 1

Measurement of *in vitro* antimicrobial activity of Active Compounds (I) against pathogenic bacteria derived from animals (Part 1):

Test microorganisms listed in Table 2 were cultured overnight in heart infusion nutrient broth (produced by Eiken Chemical Co., Ltd.). Culture broth containing about $10^8$/ml cells was diluted to a volume of 100 times. An aliquot (about 0.05 ml) of the diluted broth was inoculated onto a Mueller Hinton agar plate (produced by Eiken Chemical Co., Ltd.) to which Compound No. 1 was added in a prescribed concentration. After incubation at 37°C for 18-24 hours, the minimum inhibitory concentration (MIC), i.e., the minimum concentration of Compound No. 1 inhibiting the growth of the inoculated cells, was determined. The results are shown in Table 2.

TABLE 2

*in vitro* antimicrobial activity against pathogenic bacteria derived from animals (Part 1: Compound No.1)

(MIC, μg/ml)

| Animals | Tested microorganisms | Compound No. 1 | Control Compound * |
|---|---|---|---|
| Cow | *Escherichia coli* 15-4 | 0.05 | 0.1 |
| Pig | *Escherichia coli* 164 | 0.05 | 0.1 |
| Pig | *Salmonella typhimurium* 595 | 0.05 | 0.1 |
| Pig | *Bordetella bronchiseptica* AR3 | 0.2 | 0.39 |
| Chicken | *Escherichia coli* 442 | 0.025 | 0.05 |
| Chicken | *Salmonella typhimurium* 101 | 0.025 | 0.05 |
| Chicken | *Staphylococcus aureus* Aichi 2 | 0.1 | 0.2 |

* Ofloxacin (Japanese Patent Laid-open No. 184014/1985).

Table 2 shows that Compound No. 1 exhibited higher antimicrobial activities than its known racemic isomer (Ofloxacin).

Example 2

Measurement of *in vitro* antimicrobial activity of Active Compounds (I) against pathogenic bacteria derived from animals (Part 2):

MIC of Compounds (1)-(8) against various pathogenic bacteria derived from animals was determined in the same manner as in Example 1. The results are shown in Table 3.

**TABLE 3**

*In vitro* antimicrobial activity against pathogenic bacterial derived from animals (Part 2: Compounds No. 1-8)

(MIC, μg/ml)

| Animals | Tested microorganisms | Compound | | | | | | | | Control Compound * |
|---|---|---|---|---|---|---|---|---|---|---|
| | | No. 1 | No. 2 | No. 3 | No. 4 | No. 5 | No. 6 | No. 7 | No. 8 | |
| Cow | *Escherichia coli* 139 | 0.05 | 0.2 | 0.39 | 0.78 | 0.78 | 0.1 | 0.2 | 0.2 | 0.78 |
| Pig | *Escherichia coli* 881 | 0.05 | 0.1 | 0.2 | 0.39 | 0.78 | 0.1 | 0.2 | 0.2 | 0.39 |
| Pig | *Salmonella typhimurium* 569 | 0.05 | 0.1 | 0.39 | 0.78 | 0.78 | 0.1 | 0.2 | 0.2 | 0.39 |
| Pig | *Bordetella bronchiseptica* OKM-1 | 0.2 | 0.2 | 0.78 | 0.78 | 0.78 | 0.2 | 0.39 | 0.39 | 1.56 |
| Pig | *Pasteurella multocida* DB-12-1 | - | 0.05 | 0.1 | 0.2 | 0.1 | - | - | - | 0.2 |
| Chicken | *Escherichia coli* S6W | 0.025 | 0.1 | 0.39 | 0.78 | 0.78 | 0.1 | 0.2 | 0.2 | 0.39 |
| Chicken | *Salmonella typhimurium* 103 | 0.05 | 0.2 | 0.78 | 0.78 | 1.56 | 0.1 | 0.2 | 0.2 | 0.78 |
| Chicken | *Staphylococcus aureus* Yamaguchi 6 | 0.05 | 0.39 | 0.78 | 0.78 | 0.78 | 0.2 | 0.2 | 0.39 | 3.13 |

* Oxolinic acid

As evident from Table 3, Compound Nos. 1-8 exhibited higher *in vitro* antimicrobial activities than oxolinic acid which is widely used as an antimicrobial agent for animals.

## Claims

**Claims for the following Contracting States : DE, FR, GB, IT, NL**

**1.** Use of 3S-9-fluoro-2,3-dihydro-3-methyl-10-(4-ethyl-1-piperazinyl)-7-oxo-7H-pyrido[1,2,3-de][1,4]-benzoxazine-6-carboxylic acid or a salt or a hydrate thereof, in the manufacture of a medicament for treating or preventing infectious diseases of animals.

2. Use of Claim 1, wherein said disease is selected from the group consisting of bovine E. coli infection, bovine Salmonella infection, bovine Mycoplasma infection, bovine hemorrhagic septicemia, bovine contiguous pleuropneumonia, bovine mastitis, porcine E. coli infection, porcine Salmonella infection, porcine Pasteurella infection, porcine Mycoplasma infection, porcine atrophic rhinitis, porcine exudative epidermitis, avian E. coli infection, chicken pullorum, avian paratyphoid, avian cholera, avian infectious coryza, avian staphylococcus infection, avian Mycoplasma infection, canine E. coli septicemia, canine Salmonella infection, canine hemorrhagic septicemia, canine uterus empyema, canine cystitis, feline pleurisy, feline cystitis, feline Haemophilus infection, feline diarrhea, and feline Mycoplasma infection.

**Claims for the following Contracting States : ES, GR**

1. Use of 3S-9-fluoro-2,3-dihydro-3-methyl-10-(4-ethyl-1-piperazinyl)-7-oxo-7H-pyrido[1,2,3-de][1,4]-benzoxazine-6-carboxylic acid or a salt or a hydrate thereof, in the manufacture of a medicament for treating or preventing infectious diseases of animals. Said medicament being applicable only to animals but not to human.

2. Use of Claim 1, wherein said disease is selected from the group consisting of bovine E. coli infection, bovine Salmonella infection, bovine Mycoplasma infection, bovine hemorrhagic septicemia, bovine contiguous plueropneumonia, bovine mastitis, porcine E. coli infection, porcine Salmonella infection, porcine Pasteurella infection, porcine Mycoplasma infection, porcine atrophic rhinitis, porcine exudative epidermitis, avian E. coli infection, chicken pullorum, avian paratyphoid, avian cholera, avian infectious coryza, avian staphylococcus infection, avian Mycoplasma infection, canine E. coli septicemia, canine Salmonella infection, canine hemorrhagic septicemia, canine uterus empyema, canine cystitis, feline pleurisy, feline cystitis, feline Haemophilus infection, feline diarrhea, and feline Mycoplasma infection.

**Patentansprüche**
**Patentansprüche für folgende Vertragsstaaten : DE, FR, GB, IT, NL**

1. Verwendung von 3S-9-Fluoro-2,3-dihydro-3-methyl-10-(4-ethyl-1-piperazinyl)-7-oxo-7H-pyrido[1,2,3-de]-[1,4]benzoxazin-6-carbonsäure oder ein Salz oder ein Hydrat davon, bei der Herstellung eines Medikaments zur Behandlung oder Prävention von Infektionskrankheiten bei Tieren.

2. Verwendung gemäß Anspruch 1, worin die besagte Krankheit ausgewählt ist aus der Gruppe, bestehend aus *E. coli*-Infektionen bei Rindern, *Salmonella*-Infektionen bei Rindern, *Mycoplasma*-Infektionen bei Rindern, Septikämie bei Rindern, Pleuropneumonie bei Rindern, Mastitis bei Rindern, *E. coli*-Infektionen bei Schweinen, *Salmonella*-Infektionen bei Schweinen, *Pasteurella*-Infektionen bei Schweinen, *Mycoplasma*–Infektionen bei Schweinen, atrophische Rhinitis bei Schweinen, exsudative Epidermitis bei Schweinen, *E. coli*–Infektionen bei Geflügel, Pullorum bei Hühnern, Paratyphus bei Geflügel, Cholera bei Geflügel, ansteckende Koryza bei Geflügel, *Staphylococcus*-Infektionen bei Geflügel, *Mycoplasma*-Infektionen bei Geflügel, *E. coli*-Septikämie bei Hunden, *Salmonella*-Infektionen bei Hunden, Septikämie bei Hunden, Uterusempyema bei Hunden, Cystitis bei Hunden, Pleuritis bei Katzen, Cystitis bei Katzen, *Haemophilus*-Infektionen bei Katzen, Durchfall bei Katzen und *Mycoplasma*–Infektionen bei Katzen.

**Patentansprüche für folgende Vertragsstaaten : ES, GR**

1. Verwendung von 3S-9-Fluoro-2,3-dihydro-3-methyl-10-(4-ethyl-1-piperazinyl)-7-oxo-7H-pyrido[1,2,3-de]-[1,4]benzoxazin-6-carbonsäure oder ein Salz oder ein Hydrat davon, bei der Herstellung eines Medikaments zur Behandlung oder Prävention von ansteckenden Krankheiten bei Tieren, wobei dieses Medikament nur zur Anwendung bei Tieren aber nicht beim Menschen vorgesehen ist.

2. Verwendung gemäß Anspruch 1, worin die besagte Krankheit ausgewählt ist aus der Gruppe, bestehend aus *E. coli*-Infektionen bei Rindern, *Salmonella*–Infektionen bei Rindern, *Mycoplasma*-Infektionen bei Rindern, Septikämie bei Rindern, Pleuropneumonie bei Rindern, Mastitis bei Rindern, *E. coli*-Infektionen bei Schweinen, *Salmonella*-Infektionen bei Schweinen, *Pasteurella*-Infektionen bei Schweinen, *Mycoplasma*-Infektionen bei Schweinen, atrophische Rhinitis bei Schweinen, exsudative Epidermitis bei Schweinen, *E. coli*-Infektionen bei Geflügel, Pullorum bei Hühnern, Paratyphus bei Geflügel, Cholera bei Geflügel, ansteckende Koryza bei Geflügel, *Staphylococcus*-Infektionen bei Geflügel,

*Mycoplasma*-Infektionen bei Geflügel, *E. coli*-Septikämie bei Hunden, *Salmonella*-Infektionen bei Hunden, Septikämie bei Hunden, Uterusempyema bei Hunden, Cystitis bei Hunden, Pleuritis bei Katzen, Cystitis bei Katzen, *Haemophilus*-Infektionen bei Katzen, Durchfall bei Katzen und *Mycoplasma*-Infektionen bei Katzen.

**Revendications**

**Revendications pour les Etats contractants suivants : DE, FR, GB, IT, NL**

**1.** Utilisation de l'acide 3S-9-fluoro-2,3-dihydro-3-méthyl-10-(4-éthyl-1-pipérazinyl)-7-oxo-7H-pyrido[1,2,3-de][1,4]benzoxazine-6-carboxylique ou d'un sel ou d'un hydrate de ce composé, dans la fabrication d'un médicament destiné au traitement ou à la prévention des maladies infectieuses des animaux.

**2.** Utilisation selon la revendication 1, dans laquelle ladite maladie est choisie dans le groupe constitué par l'infection bovine due à E. coli, l'infection bovine due à Salmonella, l'infection bovine due à Mycoplasma, la septicémie hémorragique bovine, la pleuropneumonie contiguë bovine, la mastite bovine, l'infection porcine due à E. coli, l'infection porcine due à Salmonella, l'infection porcine due à Pasteurella, l'infection porcine due à Mycoplasma, la rhinite atrophique porcine, l'épidermite exsudative porcine, l'infection aviaire due à E. coli, la pullorose du poulet, la paratyphoïde aviaire, le choléra aviaire, le coryza infectieux aviaire, l'infection aviaire due aux staphylocoques, l'infection aviaire due à Mycoplasma, la septicémie canine due à E. coli, l'infection canine due à Salmonella, la septicémie hémorragique canine, l'empyème canin de l'utérus, la cystite canine, la pleurésie féline, la cystite féline, l'infection féline due à Haemophilus, la diarrhée féline, et l'infection féline due à Mycoplasma.

**Revendications pour les Etats contractants suivants : ES, GR**

**1.** Utilisation de l'acide 3S-9-fluoro-2,3-dihydro-3-méthyl-10-(4-éthyl-1-pipérazinyl)-7-oxo-7H-pyrido[1,2,3-de][1,4]benzoxazine-6-carboxylique ou d'un sel ou d'un hydrate de ce composé, dans la fabrication d'un médicament destiné au traitement ou à la prévention des maladies infectieuses des animaux, ledit médicament étant applicable uniquement aux animaux mais non à l'homme.

**2.** Utilisation selon la revendication 1, dans laquelle ladite maladie est choisie dans le groupe constitué par l'infection bovine due à E. coli, l'infection bovine due à Salmonella, l'infection bovine due à Mycoplasma, la septicémie hémorragique bovine, la pleuropneumonie contiguë bovine, la mastite bovine, l'infection porcine due à E. coli, l'infection porcine due à Salmonella, l'infection porcine due à Pasteurella, l'infection porcine due à Mycoplasma, la rhinite atrophique porcine, l'épidermite exsudative porcine, l'infection aviaire due à E. coli, la pullorose du poulet, la paratyphoïde aviaire, le choléra aviaire, le coryza infectieux aviaire, l'infection aviaire due aux staphylocoques, l'infection aviaire due à Mycoplasma, la septicémie canine due à E. coli, l'infection canine due à Salmonella, la septicémie hémorragique canine, l'empyème canin de l'utérus, la cystite canine, la pleurésie féline, la cystite féline, l'infection féline due à Haemophilus, la diarrhée féline, et l'infection féline due à Mycoplasma.